# EUROPÄISCHE PATENTANMELDUNG

(11) **EP 0 604 789 A1**
(43) Veröffentlichungstag der Anmeldung: **06.07.1994**
(21) Anmeldenummer: 93119626.5
(22) Anmeldetag: 06.12.1993
(51) Int. Cl.: A61B 17/04, A61B 17/11

(54) **Für chirurgische Zwecke bestimmtes Klemmelement zur Herstellung einer Tabaksbeutelnaht**

(30) Priorität: 31.12.1992 CH 3997/92; 05.08.1993 CH 2340/93
(71) Anmelder: K. WIDMANN AG, CH-8964 Rudolfstetten (CH)
(72) Erfinder: Schöb, Othmar, Dr. med., CH-8902 Urdorf (CH); Spillmann, Max, CH-8964 Rudolfstetten (CH)
(74) Vertreter: Fillinger, Peter, Dr.

(57) **Zusammenfassung**

Das Klemmelement weist zwei in eine Offen- und eine Schliessstellung bewegbare Klemmbacken (9, 9') auf, welche je eine längsverlaufende Nadelführung (18') aufweisen, die, wenn die Klemmbacken (9, 9') ihre Schliessstellung einnehmen für den Fadendurchzug miteinander verbunden sind. Zur Beschleunigung des Vorganges und zur exakteren Ausführung der Tabaksbeutelnaht wird vorgeschlagen, dass die Klemmbacken (9, 9') beweglich an einem Tragelement (1) gelagert sind, welches ein Nadelmagazin (14) zur Aufnahme eines Nadelpaares (16) aufweist, und dass ein Betätigungselement (3, 4, 26) vorhanden ist, um ein Nadelpaar (16) in die Nadelführungen (18') zu schieben, wenn die Klemmbacken (9, 9') ihre Schliessstellung einnehmen.

## Beschreibung

Die vorliegende Erfindung bezieht sich auf ein Klemmelement gemäss dem Oberbegriff des Anspruchs 1.

Solche Klemmelemente sind in der Chirurgie für das Operieren bei offener Bauch- oder Brusthöhle als Werkzeug zur Anfertigung einer Tabaksbeutelnaht beim Anlegen von Anastomosen und für die Durchführung von Resektionen an viszeralen Organen bekannt. Sie haben den Nachteil, dass sie beim Anlegen einer Tabaksbeutelnaht keinen wesentlichen Zeitgewinn bringen. Der Operateur muss die Nadeln des Nadelpaares einzeln in die Nadelführungen hinein und durch diese hindurch schieben. Danach wird der Faden festgezurrt und das Organ eingeschnürt. Dieser Vorgang erfordert trotz der durch das Klemmelement bedingten Arbeitserleichterung noch Geschicklichkeit und Zeit.

Die vorliegende Erfindung stellt sich die Aufgabe den genannten Nachteil zu beseitigen.

Erfindungsgemäss wird diese Aufgabe gelöst durch die kennzeichnenden Merkmale des Anspruchs 1.

Ist der abgeschnürte Organteil wegzuschneiden, ergibt sich beim bekannten Klemmelement weiter der Nachteil, dass der Abstand des Wundrandes zur Naht nicht gleichmässig ist.

Dieser Nachteil lässt sich durch die Merkmale der Ansprüche 15 und 16 beseitigen.

Die Merkmale der Ansprüche 2, 3 und 4 erlauben eine einfache konstruktive Gestaltung des Gerätes und bedingen eine leichte und einfache Handhabbarkeit unter operativen Bedingungen.

Ein weiterer Nachteil des bekannten Klemmelementes besteht darin, dass es in der laparoskopischen Chirurgie nicht einsetzbar ist. Dieser Nachteil wird beseitigt durch die Merkmale des Anspruchs 5, wobei die Merkmale der Ansprüche 6, 7 und 8 eine konstruktiv einfache und raumsparende Betätigungseinrichtung für die Klemmbacken ermöglichen.

Durch die Merkmale der Ansprüche 10 und 11 ist es möglich, die Betätigungselemente für die Klemmbacken und die Nadeln am rückwärtigen Ende des Tragrohrs anzubringen und damit das Werkzeug für seinen Einsatz in der laparoskopischen Chirurgie vergleichsweise kurz und handlich zu gestalten.

Durch die Merkmale des Anspruchs 12 ist es möglich, beim Einsatz des Werkzeuges in der laparoskopischen Chirurgie handelsübliche Trokars zu verwenden.

Durch die Merkmale der Ansprüche 13 und 14 wird eine äusserst genaue Fadenführung in der Tabaksbeutelnaht erreicht und zudem kann das Werkzeug durch die Wahl von Matrizen mit geeigneter Zahnung an den optimal auf das zu behandelnde Organ abgestimmt werden.

Das für die laparoskopische Chirurgie gestaltete Werkzeug kann durch die Merkmale der Ansprüche 17 und 18 mit einem Schneidelement für eine Resektion ausgerüstet werden, was nebst einer Beschleunigung des Vorganges den Vorteil eines definierten Wundrandabstandes zur Naht hat.

Anhand der beiliegenden schematischen Zeichnung wird die Erfindung beispielsweise erläutert. Es zeigen:
- Fig. 1: einen Längsschnitt durch ein Ausführungsbeispiel des Werkzeugs, wobei die Fig. 1A den vorderen und Fig. 1B den hinteren Teil des Werkzeugs in einer ersten Arbeitsstellung zeigt,
- Fig. 2: eine Ansicht des Verschlussstückes mit der Nadelführung in Richtung des Pfeiles II in Fig. 1,
- Fig. 3: eine gleiche Darstellung wie Fig. 1A, wobei das Werkzeug eine zweite Arbeitsstellung einnimmt,
- Fig. 4: eine gleiche Darstellung wie Fig. 1A, wobei das Werkzeug eine dritte Arbeitsstellung einnimmt,
- Fig. 5: eine Ansicht von Fig. 4 in Richtung des Pfeiles V,
- Fig. 6: eine Draufsicht auf den vorderen Teil von Fig. 4 in Richtung des Pfeiles VI,
- Fig. 7: eine Darstellung, welche das Einsetzen eines Schneidelementes zeigt und
- Fig. 8: eine an einem visceralen Organ angebrachte Tabaksbeutelnaht.
- Fig. 9: eine Seitenansicht einer Nadellafette,
- Fig.10: eine Draufsicht auf eines der Enden der Nadellafette in Richtung des Pfeiles X in Fig. 9 und
- Fig.11: eine Vorderansicht der Nadellafette in Richtung des Pfeiles XI in Fig. 9 in vergrösserter Darstellung.

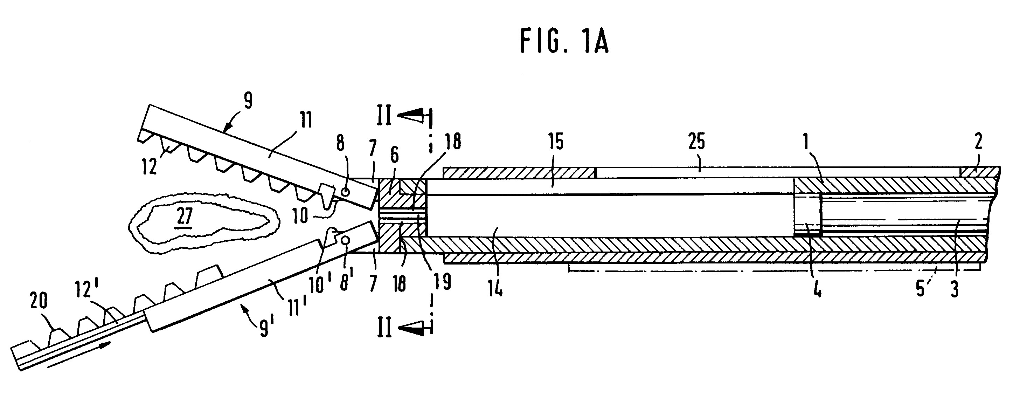

Tragendes Element des Werkzeugs ist ein ringzylindrisches Tragrohr 1. Auf dessen Aussenseite ist ein Verschlussrohr 2 und auf dessen Innenseite ist ein mit einer Kolbenstange 3 verbundener Kolben 4 achsial verschiebbar gelagert. Auf das Verschlussrohr 2 ist weiter eine im Querschnitt ringsegmentförmige Schale 5 (in Fig. 1A strichpunktiert gezeichnet) aufgesetzt. Die Schale 5 umgreift mit Reibschluss das Verschlussrohr 2 um mehr als 120° und ist um dieses dreh- und in jeder Drehlage feststellbar.

In das vordere Ende des Tragrohrs 1 ist ein Verschlussstück 6 fest eingesetzt. Dessen zylindrische Mantelflächenabschnitte sind bündig mit den Zylinderflächen des Tragrohrs 1.

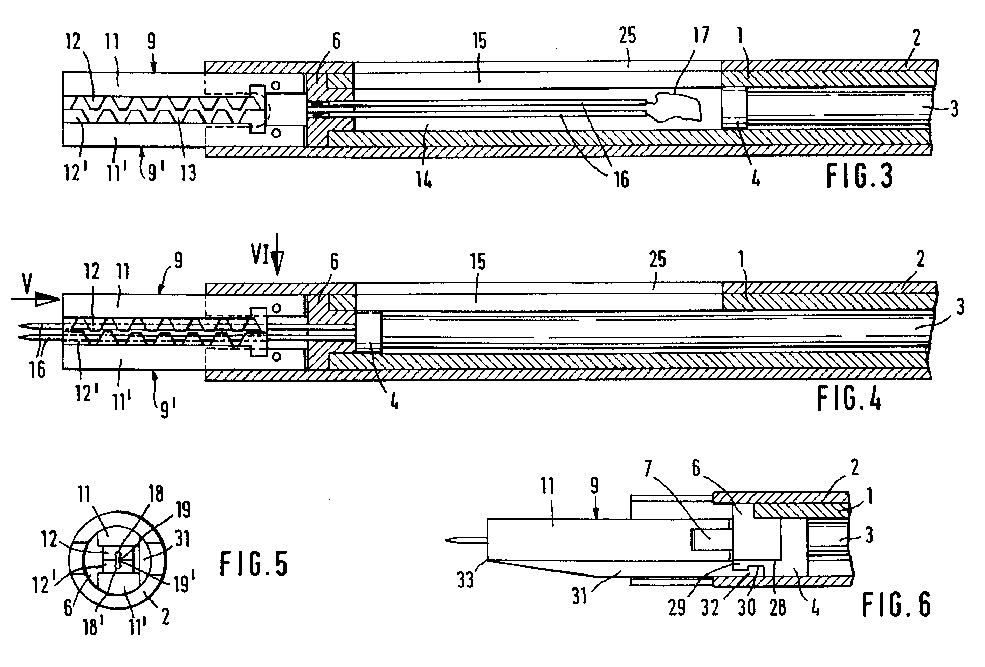

Das Verschlussstück 6 ist mit zwei nach vorne gerichteten Lagern 7 versehen, an denen je eine Klemmbacke 9, 9' um eine Achse 8, 8' schwenkbar gelagert ist. Die Klemmbacken 9, 9' werden durch achsial auf die Achsen 8, 8' aufgesetzte, vorgespannte Schraubenfedern 10, 10' in ihrer in Fig. 1A aufgeklappten Stellung gehalten und gegen deren Wirkung geschlossen. Die Klemmbacken 9, 9' sind zweiteilig und bestehen aus einer starren, an der Achse 8 bzw. 8' angelenkten, U-Profil-förmigen Schiene 11 bzw. 11', die eine T- oder V-Nut bilden, in welche je eine gezahnte Matrize 12, 12' als Backenfutter einschiebbar ist. Die eingeschobene Matrize 12, 12' ist mit der Schiene 11, 11' fest aber lösbar verbunden. Die Verbindung kann form- und/oder kraftschlüssig sein. Sind die Matrizen 12, 12' in die Schienen 11, 11' vollständig eingeschoben und sind die Klemmbacken 9, 9' geschlossen (Fig. 3), liegt jedem Zahn der einen Matrize 12, 12' eine Ausnehmung in der anderen Matrize gegenüber, so dass zwischen den Matrizen 12, 12' ein zusammenhängender zickzackförmiger Zwischenraum 13 besteht. Damit sich im Bereich der Zähne die Klemmflächen 20 nicht verzahnen, sind die vordersten und hintersten Zähne der Matrizen 12, 12' als Anschläge gestaltet.

Das Tragrohr 1 weist zwischen dem in der hinteren Endlage befindlichen Kolben 4 und dem Verschlussstück 6 ein Nadelmagazin 14 auf, in welches durch einen Schlitz 15 ein mit den Enden eines Fadens 17 verbundenes Nadelpaar 16 einlegbar ist (Fig. 3). Zur längsachsialen Führung der Nadeln 16 werden deren Spitzen in zwei entsprechende Bohrungen 18 im Verschlussstück 6 gesteckt, welche durch einen Schlitz 19 miteinander verbunden sind.

Jede der Bohrungen 18 fluchtet mit einer entsprechenden Bohrung 18' in einer der beiden Matrizen 12, 12', welche Bohrungen 18' gegen die Klemmflächen 20 offen sind. Wie die Bohrungen 18 durch den Schlitz 19 sind dadurch bei geschlossenen Klemmbacken 9, 9' die Bohrungen 18' durch einen Schlitz 19' verbunden (Fig. 5).

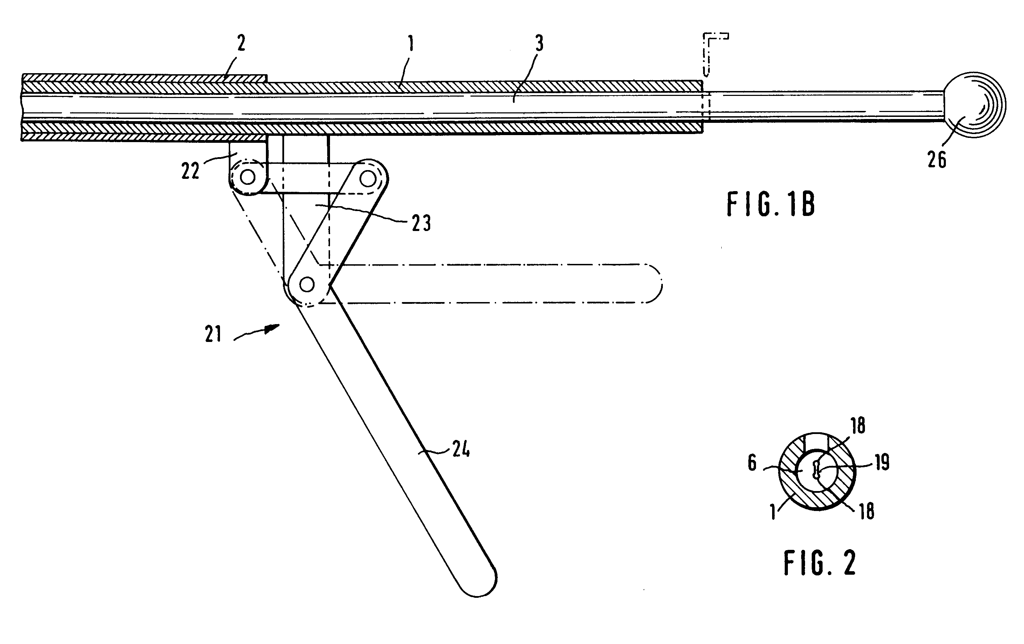

Das Verschlussrohr 2 ist mittels eines Hebelmechanismus 21 (Fig. 1B) zwischen einer hinteren (Fig. 1A) und einer vorderen Endlage (Fig. 3 und 4) achsial auf dem Tragrohr 1 verschiebbar. Der Hebelmechanismus 21 ist einerseits an einer mit dem Verschlussrohr 2 fest verbundenen Lasche 22 und anderseits an einer mit dem Tragrohr 1 fest verbundenen Lasche 23 angelenkt. Ist ein Betätigungshebel 24 des Hebelmechanismus 21 nach unten geschwenkt (in Fig. 1B mit ausgezogenen Strichen gezeigt) ist das Verschlussrohr 2 in die hintere Endlage gezogen. Wird es nach oben verschwenkt (gestrichelt gezeichnet) verschiebt sich das Verschlussrohr 2 in seine vordere Endlage (Fig. 3 und 4), wo ein im Verschlussrohr 2 angebrachter Schlitz 25 mit dem Schlitz 15 des Tragrohrs fluchtet, so dass das Nadelmagazin 14 für das Einlegen der Nadeln 16 mit dem Faden 17 frei zugänglich ist. Nach dem Einlegen der Nadeln 16 wird das Nadelmagazin 14 durch ein Drehen der Schale 5 geschlossen.

Die Kolbenstange 3 weist an ihrem rückwärtigen Ende einen kugeligen Handgriff 26 für das Verschieben des Kolbens 4 im Tragrohr 1 auf.

Das Verschlussrohr 2 ist vorzugsweise gegen das Tragrohr 1 und das Tragrohr 1 gegen die Kolbenstange 3 mit einer nicht dargestellten Ringdichtung abgedichtet, wodurch, wenn das Werkzeug in die Bauch- oder Brusthöhle eingeführt ist, ein Abbau des Überdrucks verhindert wird.

Das beschriebene Werkzeug funktioniert wie folgt.

Vorerst werden die dem zu behandelnden Organ entsprechenden, das heisst, mit gröberer oder feinerer Zahnung versehenen Matrizen 12, 12' in die Klemmbacken 9, 9' eingeschoben. Danach wird der Hebel 21 hochgeklappt und damit das Verschlussrohr 2 nach vorne über die Klemmbacken 9, 9' geschoben. Die Klemmbacken 9, 9' werden dabei geschlossen. Alsdann wird der Handgriff 26 nach hinten bewegt und der Kolben 4 aus dem Bereich des Nadelmagazins 14 gezogen. Nun wird die Schale 5 gedreht, bis das Nadelmagazin 14 frei zugänglich ist, worauf ein Nadelpaar 16 mit einem Faden 17 eingelegt wird und die Nadelspitzen in die Bohrungen 18 gesteckt werden. Nach dem Schliessen des Nadelmagazins 14 mit der Schale 5 ist das Werkzeug einsatzbereit und kann durch einen Trokar in die Bauch- oder Brusthöhle eingeführt werden.

Nach dem Einführen der Klemmbacken 9, 9' in die Bauchhöhle werden sie geöffnet indem der Hebel 24 nach unten verschwenkt und das Verschlussrohr 2 nach hinten gezogen wird. Dieses gibt dabei die Klemmbacken 9, 9' frei, die sich unter der Wirkung der Federn 10 öffnen. Nun kann das Werkzeug im Trokar verschoben werden, bis sich die gewünschte Stelle des zu behandelnden Organs 27 (Fig. 1A) zwischen den Klemmbacken 9, 9' befindet. Nimmt das Werkzeug diese Stellung ein, wird der Hebel 24 hoch geschwenkt, das heisst, die Klemmbacken 9, 9' werden wieder geschlossen und die Nahtstelle des Organs wird zwischen den Matrizen 12, 12' eingeklemmt. Danach wird der Kolben 4 nach Lösen einer Arretierung 4 mittels des Handgriffs 26 nach vorne vorgeschoben, wobei er die Nadeln 16 durch die Matrizen 12, 12' und damit durch die zwei in Zickzackfaltung aneinanderliegenden Wände des Organs 27 stösst, bis die Nadelspitzen über das freie Ende der Klemmbacken 9, 9' vorstehen. Anschliessend wird der Kolben 4 sofort wieder zurückgezogen, um ein Einklemmen des Fadens 17 zu vermeiden und um ein allfällig eingebautes (später beschriebenes) Schneidelement im Verschlussrohr 2 zu positionieren.

Die Nadeln 16 werden alsdann mittels eines Werkzeugs an den Spitzen erfasst und vollständig aus den Klemmbacken 9, 9' herausgezogen. Anschliessend werden die Klemmbacken 9, 9' durch ein Verschwenken des Hebels 24 nach unten wieder geöffnet und das Werkzeug vom Organ 27 entfernt. Durch ein Festzurren des Fadens 17 kann nun das Organ 27 nach Art eines Tabaksbeutelverschlusses zusammengezogen und verschlossen werden. Der abgeschnürte Organteil kann danach mit einem Skalpell vom restlichen Organ abgetrennt werden. Zur Beschleunigung und Erleichterung dieses Vorganges kann, wie nachfolgend mit Bezug auf die Fig. 5 bis 8 beschrieben, das Werkzeug mit einem Schneidelement ausgerüstet werden.

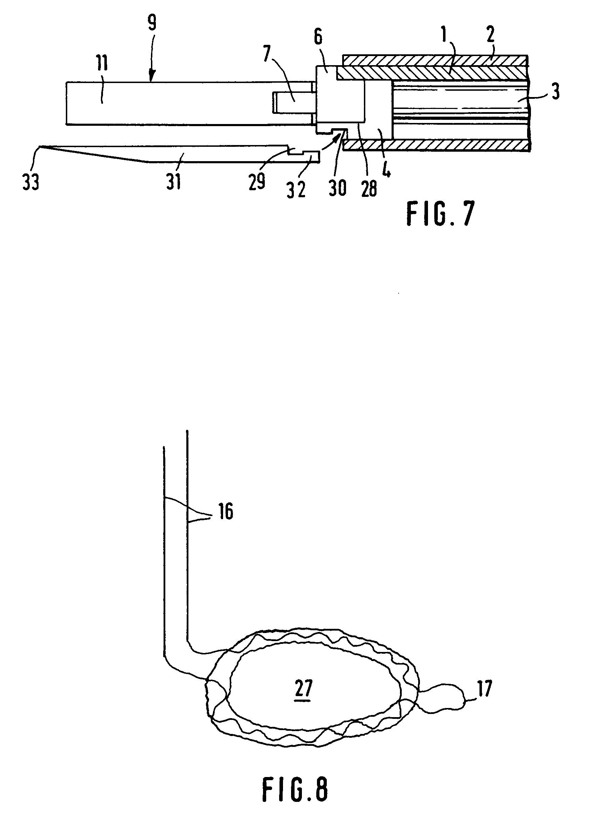

Gemäss den Fig. 5 bis 7 weist das Verschlussstück 6 einen kreissegmentförmigen Ausschnitt 28 auf, in den ein mit dem Kolben 4 fest verbundener Fortsatz 29 entsprechenden Querschnitts schiebbar ist. Der Fortsatz 29 ist mit einer Hinterschneidung 30 versehen, an welcher ein Schneidelement 31 mit einem komplementär gestalteten Verbindungsteil 32 formschlüssig einhängbar ist. Das Schneidelement 31 ist am freien Ende mit einer Schneide 33 versehen und weist, wie Fig. 5 zeigt, einen kreissegmentförmigen Querschnitt auf. Es wird durch die formschlüssige Verbindung mit dem Kolben 4 stets mit diesem hin und her bewegt und ist dabei einerseits an den Schienen 11, 11' der Klemmbacken 9, 9' und anderseits an der Innenwand des Verschlussrohres 2 geführt. Das Schneidelement 31 kann, wie Fig. 7 zeigt, in die Hinterschneidung 30 eingehängt (bzw. von dieser gelöst) werden, wenn das Verschlussrohr 2 in die hintere Endlage verschoben ist. Ist das Schneidelement 31 in das Werkzeug eingesetzt, wird jeweils, wenn während eines Kolbenvorschubs die Nadeln 16 durch die Matrizen 12, 12' bzw. durch die aneinander liegenden Organwände gestossen werden, gleichzeitig der abzutrennende Organteil vom im Körper verbleibenden Organ abgeschnitten. Diese Ausführungsform hat nebst einem Zeitgewinn den Vorteil, dass der Trennschnitt in einem stets gleichbleibenden, definierten Abstand von der Naht erfolgt.

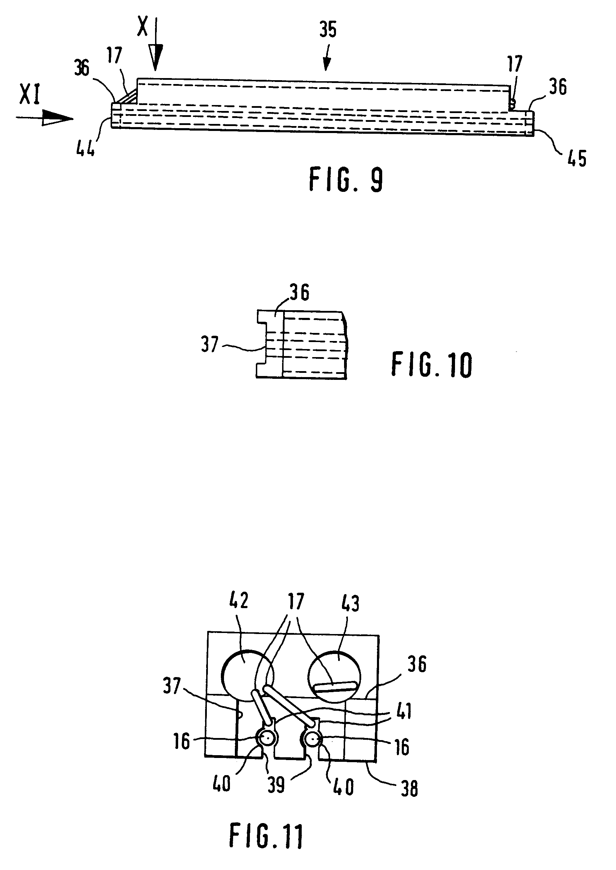

Beim Einlegen des Nadelpaares 16 und des Fadens 17 in das Nadelmagazin 14 ist darauf zu achten, dass der Faden 17 nicht verknotet oder hängenbleibt, wenn das Nadelpaar 16 durch den Kolben 4 ausgestossen wird. Um dies zu vermeiden und um das Instrument rascher einsatzbereit zu haben, ist die Verwendung einer Nadellafette gemäss den Fig. 9 bis 11 zweckmässig. Diese wird dem Chirurgen bestückt mit Nadeln und Faden als sterile Einwegpackung angeliefert. Die Nadellafette 35 ist ein länglicher, im Querschnitt rechteckiger Kunststoffkörper, der an beiden Enden mit einer Absetzung 36 und zudem mit einer Ausnehmung 37 versehen ist. In die Unterseite 38 des Körpers öffnen sich zwei achsparallele Längsschlitze 39, deren Flanken auf etwa halber Höhe durch eine zylindrische Ausweitung ein Nadellager 40 bilden. Der über dem Nadellager 40 befindliche Teil des Schlitzes 39 bildet einen Fadenkanal 41. Parallel über den Schlitzen 39 ist der Körper von zylindrischen Bohrungen 42, 43 durchsetzt, die als Fadenkammern den überwiegenden Teil des Fadens 17 aufnehmen. Zur Bestückung der Nadellafette mit dem an ihren Enden durch den Faden 17 verbundenen Nadelpaar 16 werden die Nadeln 16 mit ihrer Rückseite voran an der Vorderseite 44 des Körpers je in eines der Nadellager 40 eingeschoben und zwar so, dass die von den Nadelenden weglaufenden Fadenenden über den Nadeln 16 in den entsprechenden Fadenkanal 41 zu liegen kommen. Ist das Nadelpaar 16 vollständig in den Körper eingeschoben, bildet der Faden 17 an der Körpervorderseite 44 eine grosse Schlaufe, die von einem Fadenkanal 41 zum anderen führt. Diese Fadenschlaufe wird nun mit der Schlaufenmitte voran durch die Fadenkammer 42 geschoben und rückseitig herausgezogen, so dass nun an der Körperrückseite 45 eine kleinere Fadenschlaufe über die Fadenkammer 42 vorsteht. Diese kleinere Fadenschlaufe wird nun mit ihrer Mitte voran von der Rückseite 45 her in die zweite Fadenkammer 43 eingeschoben und darin vollständig verstaut. Die solcher Art bestückte Nadellafette wird alsdann mit der Vorderseite 44 voran in das Nadelmagazin 14 eingelegt, wobei das Nadelmagazin in seiner Querschnittsform so ausgelegt ist, dass die Nadellager 40 koachsial zu den Bohrungen 18 ausgerichtet sind. Dabei ist der Querschnitt des Kolbens 4 so auszubilden, dass er während des Vorschubes an der Nadellafette vorbei gleiten kann und gleichzeitig mit zwei seitab stehenden Nocken in die Schlitze 39 eingreift und dabei das Nadelpaar 16 ausstösst. Die Länge der Nocken ist dabei so bemessen, dass sie die Nadelenden hintergreifen ohne in die Farbkanäle 41 hineinzuragen. Die Ausnehmung 37 und die Absetzung 36 bieten während des Einschubs des Nadelpaares 16 Gewähr, dass der Faden 17 nicht zwischen der Stirnwand des Nadelmagazins 14 und der Vorderseite 44 der Nadellafette eingeklemmt wird. Die gleiche Ausbildung von Vorder- und Rückseite 44, 45 der Nadellafette hat beim Beschicken den Vorteil, dass das Nadelpaar 16 nicht von einer falschen Seite in das Nadellager 40 eingeschoben werden kann.

## Patentansprüche

1. Für chirurgische Zwecke bestimmtes Klemmelement zur Herstellung einer Tabaksbeutelnaht an einem viszeralen Organ (27) mit zwei in eine Offen- und eine Schliessstellung bewegbaren Klemmbacken (9, 9'), welche je eine längsverlaufende Nadelführung (18') aufweisen, die, wenn die Klemmbacken (9, 9') ihre Schliessstellung einnehmen für den Fadendurchzug miteinander verbunden sind, dadurch gekennzeichnet, dass die Klemmbacken (9, 9') beweglich an einem Tragelement (1) gelagert sind, welches ein Nadelmagazin (14) zur Aufnahme eines Nadelpaares (16) aufweist, und dass ein Betätigungselement (3, 4, 26) vorhanden ist, um ein Nadelpaar (16) in die Nadelführungen (18') zu schieben, wenn die Klemmbacken (9, 9') ihre Schliessstellung einnehmen.

2. Klemmelement nach Anspruch 1, dadurch gekennzeichnet, dass das Tragelement (1) ein Tragrohr ist, an dessen einem Ende die Klemmbacken (9, 9') beweglich gelagert sind, und dass das Nadelmagazin (14) im Tragrohr (1) an dem den Klemmbacken (9, 9') benachbarten Ende angeordnet und durch eine Öffnung (15) im Tragrohrmantel von aussen zugänglich ist.

3. Klemmelement nach Anspruch 2, dadurch gekennzeichnet, dass im Tragrohr (1) ein Kolben (4) achsial verschiebbar gelagert ist, der beim Vorlauf ein im Nadelmagazin befindliches Nadelpaar (16) in die Nadelführungen (18') der die Schliessstellung einnehmenden Klemmbacken (9, 9') schiebt und im Rücklauf das Nadelmagazin (14) frei gibt.

4. Klemmelement nach Anspruch 2 oder 3, dadurch gekennzeichnet, dass die Klemmbacken (9, 9') symmetrisch mit Bezug auf die Längsachse des Tragrohrs (1) beweglich sind, dass die Nadelführungen (18') in der Schliessstellung der Klemmbacken (9, 9') achsparallel zum Tragrohr (1) ausgerichtet sind, und dass im Tragrohr (1) zwischen dem Nadelmagazin (14) und den Nadelführungen (18') mit diesen fluchtende und durch einen Schlitz (19) miteinander verbundene Zentrierbohrungen (18) für ein im Nadelmagazin (14) befindliches Nadelpaar (16) vorhanden sind.

5. Klemmelement nach einem der Ansprüche 2 bis 4, dadurch gekennzeichnet, dass die Klemmbacken (9, 9') am stirnseitigen Ende des Tragrohrs (1) um zur Tragrohrlängsachse quer orientierte Achsen (8, 8') schwenkbar gelagert sind.

6. Klemmelement nach Anspruch 5, dadurch gekennzeichnet, dass in der Schliessstellung der Klemmbacken (9, 9') deren der Klemmfläche abgewandten Seiten mit der Umfangsmantelfläche des Tragrohrs (1) fluchten.

7. Klemmelement nach Anspruch 6, dadurch gekennzeichnet, dass auf dem Tragrohr (1) ein Verschlussrohr (2) achsial zwischen zwei Endlagen verschiebbar gelagert ist, welches in einer ersten Endlage die Klemmbacken (9, 9') teilweise übergreift und in die Schliessstellung zwingt und in der zweiten Endlage frei gibt.

8. Klemmelement nach einem der Ansprüche 1 bis 7, dadurch gekennzeichnet, dass die Klemmbacken durch Federelemente (10) in der Offenstellung gehalten und gegen deren Wirkung in die Schliessstellung bewegbar sind.

9. Klemmelement nach einem der Ansprüche 2 bis 8, dadurch gekennzeichnet, dass im Verschlussrohr (2) eine der Öffnung (15) dem Tragrohr (1) entsprechende Öffnung (25) vorhanden ist, welche in der ersten Endlage des Verschlussrohrs (2) mit der Öffnung des Tragrohrs (1) fluchtet.

10. Klemmelement nach einem der Ansprüche 3 bis 9, dadurch gekennzeichnet, dass der Kolben (4) an einer das Tragrohr (1) rückwärts überragenden Kolbenstange (3) befestigt ist, die an das Tragrohr (1) überragenden Teil vorzugsweise einen Handgriff (26) aufweist.

11. Klemmelement nach einem der Ansprüche 7 bis 10, dadurch gekennzeichnet, dass am rückwärtigen Ende des Tragrohrs (1) ein Hebelmechanismus (21) angelenkt ist, um das Verschlussrohr (2) zwischen seinen Endlagen zu verschieben.

12. Klemmelement nach einem der Ansprüche 7 bis 11, dadurch gekennzeichnet, dass der Aussendurchmesser des Verschlussrohrs (2) höchstens 18 Millimeter beträgt.

13. Klemmelement nach einem der Ansprüche 1 bis 12, dadurch gekennzeichnet, dass die Klemmbacken (9, 9') gezahnte Klemmflächen aufweisen, die in der Schliessstellung einen zusammenhängenden, zickzackförmigen Zwischenraum (13) bilden.

14. Klemmelement nach einem der Ansprüche 1 bis 13, dadurch gekennzeichnet, dass die Klemmbacken (12, 12') zweiteilig sind und je eine Schiene (11, 11') und eine daran auswechselbar befestigte Matrize (12, 12') aufweisen.

15. Klemmelement nach einem der Ansprüche 1 bis 14, dadurch gekennzeichnet, dass es ein parallel längs den in der Schliessstellung befindlichen Klemmbacken (9, 9') verschiebbares Schneidelement (31) aufweist.

16. Klemmelement nach Anspruch 15, dadurch gekennzeichnet, dass das Schneidelement (31) mit dem Betätigungsorgan (3, 4, 26) verbunden und von diesem mit dem im Nadelmagazin (14) befindlichen Nadelpaar (16) während des Einschiebens in die Nadelführungen (18') bewegt wird.

17. Klemmelement nach einem der Ansprüche 4 bis 16, dadurch gekennzeichnet, dass der Kolben (4) einen an den Zentrierbohrungen (18) vorbei greifenden Fortsatz (29) mit einer Befestigungsstelle (30) für das Schneidelement (31) aufweist.

18. Schneidelement für ein Klemmelement nach Anspruch 17, dadurch gekennzeichnet, dass das zwischen den Schienen (11, 11') einerseits und dem Verschlussrohr (2) anderseits geführte Schneidelement (31) an der Befestigungsstelle (30) auswechselbar befestigt ist.

19. Nadellafette für ein Klemmelement nach einem der Ansprüche 1 bis 17, gekennzeichnet durch einen prismatischen Körper, der zwei achsparallele, durchgehende, nach einer Seite (38) offene Nadellager (14) aufweist, an welche seitlich ein Fadenkanal (41) anschliesst und durch wenigstens eine nach aussen offene Fadenkammer (42, 43).

20. Nadellafette nach Anspruch 19, dadurch gekennzeichnet, dass die Nadellager (40) und die Fadenkanäle (41) in eine an der Vorderseite (44) des Körpers angeordnete Ausnehmung (37) münden.

21. Nadellafette nach Anspruch 20, dadurch gekennzeichnet, dass die Fadenkammer (42, 43) gegen die Vorderseite (44) des Körpers offen ist und vorzugsweise in eine Absetzung (36) über der Ausnehmung (37) mündet.

22. Nadellafette nach einem der Ansprüche 19 bis 20, dadurch gekennzeichnet, dass die Vorderseite (44) und die Rückseite (45) des Körpers gleich ausgebildet sind.
